# EUROPEAN PATENT SPECIFICATION

(11) **EP 2 554 989 B1**
(45) Date of publication and mention of the grant of the patent: **16.05.2018**
(21) Application number: 11765773.4
(22) Date of filing: 31.03.2011
(51) Int. Cl.: G01N 33/543

(54) **METHOD FOR AVOIDING INFLUENCE OF ENDOGENOUS LIPOPROTEIN AND REAGENT**
VERFAHREN ZUR VERMEIDUNG DES EINFLUSSES ENDOGENER LIPOPROTEINE UND REAGENZIEN
MÉTHODE PERMETTANT D'ÉVITER L'INFLUENCE DE LIPOPROTÉINES ENDOGÈNES ET RÉACTIF

(30) Priority: 31.03.2010 JP 2010083735
(43) Date of publication of application: 06.02.2013
(73) Proprietor: Sekisui Medical Co., Ltd., Tokyo 103-0027 (JP)
(72) Inventor: ABE, Takayuki, Ryugasaki-shi Ibaraki 301-0852 (JP); TAKAHASHI, Yuki, Ryugasaki-shi Ibaraki 301-0852 (JP); OTA, Tetsuya, Ryugasaki-shi Ibaraki 301-0852 (JP)
(74) Representative: Hartz, Nikolai
(86) International application number: PCT/JP2011/058283
(87) International publication number: WO 2011/125873

(56) References cited:
- WO-A1-83/02672
- JP-A- 7 103 980
- JP-A- 2001 242 171
- JP-A- 2003 501 631
- SHUNSUKE YACHITA: 'Densensei Kikanshien HI Test ni Okeru Hitokuiteki Inhibitor Jyokyo ni Tsuite' JOURNAL OF THE JAPANESE SOCIETY ON POULTRY DISEASES vol. 20, no. 4, December 1984, pages 186 - 192

## Description

### Field of the Invention

The present invention relates to a reagent for assaying antiphospholipid antibodies and a reagent for assaying a prostate specific antigen, based on a method for avoiding any interference effect caused by an endogenous lipoprotein.

### Background of the Invention

In our bodies, antiphospholipid antibodies are produced by the following two diseases. One is syphilis, which is caused by infection with *Treponema Pallidum* as a pathogen thereof. The other is antiphospholipid antibody syndrome, which is a type of autoimmune disease such as systemic lupus erythematosus (SLE).

In both cases, the antiphospholipid antibodies are an antibody produced by a lipid antigen predominantly containing cardiolipin, which is a type of phospholipid. Thus, a test reagent employed for the diagnosis of the above disease contains cardiolipin.

Currently, several methods employing the aforementioned phospholipid are employed as syphilis test methods. Such methods include the VDRL (Veneral Disease Research Lab.) method, employing carbon or powdered kaolin as a carrier; the RPR (rapid plasma reagin) card test (Non-Patent Document 1); and the latex agglutination method which employs a latex (e.g., polystyrene copolymer latex) carrier and is conducted by a biochemical auto-analyzer (Patent Document 1).

Meanwhile, antiphospholipid antibody syndrome is detected by ELISA (Patent Document 2).

In these tests, serum, plasma, cerebrospinal fluid, and similar are used.

Prostatic cancer is a malignant tumor of men, and the incidence thereof has drastically increased, particularly in Japan and the USA. Since prostatic cancer is a slow-growing tumor and is highly susceptible to radiotherapy and anti-androgen therapy, early detection thereof is a key factor.

Prostate specific antigen (hereinafter may be referred to simply as "PSA") is a type of serine protease secreted by prostatic epithelial cells and is a glycoprotein having a molecular weight of 33,000 to 34,000 Da. Since the blood PSA level of a subject having a prostatic disease rises as compared with a healthy subject, the PSA level is a useful marker for the early detection of a prostatic disease (particularly prostatic cancer). PSA includes a complex form, which bonds to a protease inhibitor in blood, and a non-binding, free PSA (hereinafter may be referred to as "fPSA"). Generally, PSA is in the complex PSA form in blood, including prostate specific antigen-α1-anti-chymotrypsin complex (hereinafter may be referred to as "PSA-ACT"), prostate specific antigen-α2-macroglobulin complex, etc. Among these PSA species, two species, fPSA and PSA-ACT, can be detected by immunological assay.

### Prior Art Documents

### Patent Documents

Patent Document 1: JP-A-H07-103980
Patent Document 2: JP-A-H06-148193
Patent Document 3: JP-A-2001-242171

### Non-Patent Documents

Non-Patent Document 1: Public Health Reports Vol. 75 (1960), 985-988

### Summary of the Invention

### Problem to be solved by the invention

Generally, when a reagent for predicting the presence of a disease is employed, the reagent being employed in optical measurement of immune agglutination caused by antigen-antibody reaction, in some cases, measurements may be lower than the correct values, due to the influence of interfering substances present in the samples. This phenomenon can be confirmed by a considerable difference in measurement between the case in which an analyte is added to a solution such as physiological saline or buffer free from the influence of interfering substances present in the sample and the case in which the analyte is added to a sample such as serum or plasma containing an interfering substance.

This interference causes the following two problems.
(1) When the amount of antibody or antigen in a sample is low, immune agglutination might proceed insufficiently due to an interfering substance. In this case, the disease which the patient suffers may fail to be detected.
(2) When the amount of antibody or antigen in a sample is higher than the assay limit of the assay reagent, the sample is diluted with a diluent such as physiological saline or serum for accurate measurement for the accurate measurement. The amount of antibody or antigen is reduced to fall within a measurable range, and the correct amount of antibody or antigen is calculated by multiplying the dilution factor by the value obtained from the diluted sample. Since physiological saline contains no interfering substance, there is a considerable difference in the measured of antibody or antigen between the case in which the sample is diluted with serum containing no antibody and the case in which the sample is diluted with physiological saline, which hinders accurate measurements.

Thus, an object of the present invention is to identify the aforementioned interfering substance present in serum or plasma, to thereby provide means for avoiding any interference effect caused by the substance.

### Means for solving the problems

The present inventors have conducted extensive studies to identify the interfering substance present in such a serum sample or such a plasma sample. Quite surprisingly, the inventors have found that the interfering substance is an endogenous lipoprotein, from the observation that the absorbance attributed to antigen-antibody reaction lowers by addition of a lipoprotein to an assay sample based on physiological saline. A further study by the inventors has also elucidated that the influence of the endogenous lipoprotein on the measurements can be avoided by adding a glycerophospholipid to the immune reaction system, whereby an antibody or an antigen contained in the sample can be determined with higher accuracy. The present invention has been accomplished on the basis of these findings.

Accordingly, the present invention is directed to the subject matter of claim 1 to 3, in particular to the following.
(1) A reagent for assaying an antibody or an antigen serving as an analyte in blood by immune reaction with an antigen when the analyte is an antibody or by immune reaction with an antibody when the analyte is an antigen, wherein the antigen, when the analyte is an antibody, or the antibody, when the analyte is an antigen, is supported on a latex carrier formed of a synthetic polymer, and the amount of analyte is measured by optically measuring the degree of agglutination formed by the immune reaction between the latex carrier and the analyte, wherein a glycerophospholipid in the form of solution or dispersion is incorporated into the immune reaction system, wherein the glycerophospholipid is one or more species selected from the group consisting of phosphatidylcholine, phosphatidylglycerol, and phosphatidylethanolamine, and wherein the analyte is an antiphospholipid antibody or a prostate specific antigen.
(2) The reagent as described in (1) above, wherein the antiphospholipid antibody serving as an analyte is an anti-syphilis phospholipid antibody generated in blood through infection with syphilis.
(3) An assay reagent as described in (1) above, wherein the antiphospholipid antibody serving as an analyte is an antiphospholipid antibody generated in blood through antiphospholipid antibody syndrome, which is an autoimmune disease.

Patent Document 3 discloses that phosphatidylcholine is added to suppress non-specific agglutination, which is occasionally observed in negative samples, in counting immunoassay. Non-specific agglutination is an agglutination reaction other than a proper specific immunoagglutination reaction, which is caused by a substance rarely present in human blood samples (e.g., an antibody to a component contained in a reagent). In other words, non-specific agglutination is an agglutination reaction which is not an immunoreaction between antiphospholipid antibody (i.e., detection target) and phospholipid. The phosphatidylcholine disclosed in Patent Document 3 is used to prevent such reaction, and therefore, can be regarded a so-called non-specific agglutination inhibitor.

In contrast, the glycerophospholipid of the present invention is used to avoid inhibition of normal immunoagglutionation, the inhibition being caused by a lipoprotein generally contained in human blood samples, whereby a detection target (i.e., an antibody or an antigen) can be detected more accurately. That is, the glycerophospholipid can be regarded as an agent for avoiding any influence caused by lipoprotein.

The effect of glycerophospholipid of the present invention essentially differs from that of phosphatidylcholine disclosed in Patent Document 3.

### Advantageous effect of the invention

A characteristic feature of the assay reagent provided by the present invention resides in that a glycerophospholipid is incorporated into the immune reaction system, to thereby avoid the interference effect of an endogenous lipoprotein. Thus, when an assay is performed by use of a reagent for an antiphospholipid antibody measurement which is interfered by an endogenous lipoprotein, relatively accurate measurements can be obtained by use of the reagent of the present invention which can avoid the interference effect of such an endogenous lipoprotein.

### Brief Description of the Drawings

[Fig. 1] Fig. 1 is a graph showing the results of Test 1 of Comparative Example 1, with the reaction system containing no glycerophospholipid.
[Fig. 2] Fig. 2 is a graph showing the results of Test 1 of Example 2, with the reaction system containing 0.06 wt.% of hen's egg yolk-derived phosphatidylglycerol.
[Fig. 3] Fig. 3 is a graph showing the results of Test 2 of Example 1, with the reaction system containing 0.12 wt.% of hen's egg yolk-derived phosphatidylglycerol.
[Fig. 4] Fig. 4 is a graph showing the results of Test 2 of Example 2, with the reaction system containing 0.06 wt.% of hen's egg yolk-derived phosphatidylglycerol.
[Fig. 5] Fig. 5 is a graph showing the results of Test 2 of Example 3, with the reaction system containing 0.03 wt.% of hen's egg yolk-derived phosphatidylglycerol.
[Fig. 6] Fig. 6 is a graph showing the results of Test 2 of Example 4, with the reaction system containing 0.03 wt.% of a synthetic phosphatidylglycerol, 1,2-dimyristoyl-sn-glycero-3-phosphoglycerol sodium salt.
[Fig. 7] Fig. 7 is a graph showing the results of Test 2 of Example 5, with the reaction system containing 0.06 wt.% of hen's egg yolk-derived phosphatidylethanolamine.
[Fig. 8] Fig. 8 is a graph showing the results of Test 2 of Example 6, with the reaction system containing 0.03 wt.% of a synthetic phosphatidylcholine, 1,2-dimyristoyl-sn-glycero-3-phosphocholine.
[Fig. 9] Fig. 9 is a graph showing the results of Test 2 of Example 7, with the reaction system containing 0.03 wt.% of a synthetic phosphatidylcholine, 1-palmitoyl-2-stearoyl-sn-glycero-3-phosphocholine.
[Fig. 10] Fig. 10 is a graph showing the results of Test 2 of Example 8, with the reaction system containing 0.015 wt.% of hen's egg yolk-derived phosphatidylglycerol and 0.015 wt.% of hen's egg yolk-derived phosphatidylethanolamine.
[Fig. 11] Fig. 11 is a graph showing the results of Test 2 of Comparative Example 1, with the reaction system containing no glycerophospholipid.
[Fig. 12] Fig. 12 is a graph showing the results of Test 3 of Example 9, with the reaction system containing 0.015 wt.% of hen's egg yolk-derived phosphatidylglycerol.
[Fig. 13] Fig. 13 is a graph showing the results of Test 3 of Comparative Example 2, with the reaction system containing no glycerophospholipid.

### Detailed Description of the Invention

The present invention discloses a method for avoiding any interference effect caused by an endogenous lipoprotein in an assay of an analyte in blood by immune reaction by use of a reagent containing an antigen when the analyte is an antibody, or an antibody when the analyte is an antigen, wherein the method comprises incorporating a glycerophospholipid into the immune reaction system and to a reagent in which any interference effect caused by an endogenous lipoprotein is avoided.

The glycerophospholipid incorporated into the immune reaction system for avoiding any interference effect caused by an endogenous lipoprotein is phosphatidylcholine, phosphatidylglycerol, or phosphatidylethanolamine.

The glycerophospholipid is preferably incorporated into the reaction system at a concentration of 0.005 to 0.20 wt.%, more preferably 0.015 to 0.12 wt.%. However, no particular limitation is imposed on the glycerophospholipid concentration, since the amount of glycerophospholipid must be appropriately tuned depending on the amount of sample to be analyzed.

The glycerophospholipid shall be present in the reaction system such as in a sample-diluent or in a reagent containing an antibody or an antigen. The glycerophospholipid is preferably incorporated into a sample-diluent for the purpose of avoiding the influence of endogenous lipoprotein.

The phosphatidylcholine may originate from animals or plants and is generally selected from phosphatidylcholines purified from soybean or hen's egg yolk (hereinafter may be referred to simply as egg yolk).

No particular limitation is imposed on the number of carbon atoms and the unsaturation degree of each of the two acyl groups in the phosphatidylcholine. Generally, the acyl group has 10 to 22 carbon atoms and 0 to 2 unsaturated bonds. In addition, the two acyl groups are not necessarily equal in number of carbon atoms and of unsaturated bonds. The two acyl groups may be a combination of those having different numbers of carbon atoms from each other, and may be a combination of two saturated acyl groups, a combination of a saturated acyl group and an unsaturated acyl group, or a combination of two unsaturated acyl groups may be employed. Generally, phosphatidylcholines originating from animals and plants are in the form of a mixture of phosphatidylcholine species bearing acyl groups having different number of carbon atoms and different unsaturation degrees.

The aforementioned phosphatidylcholine may be a chemically synthesized product. Examples of commercial products thereof include 1,2-didecanoyl-sn-glycero-3-phosphocholine, 1,2-dimyristoyl-sn-glycero-3-phosphocholine, 1,2-dipalmitoyl-sn-glycero-3-phosphocholine, 1,2-distearoyl-sn-glycero-3-phosphocholine, 1,2-dioleoyl-sn-glycero-3-phosphocholine, 1,2-dilinoleoyl-sn-glycero-3-phosphocholine, 1,2-diercoyl-sn-glycero-3-phosphocholine, 1-myristoyl-2-palmitoyl-sn-glycero-3-phosphocholine, 1-myristoyl-2-stearoyl-sn-glycero-3-phosphocholine, 1-myristoyl-2-oleoyl-sn-glycero-3-phosphocholine, 1-palmitoyl-2-oleoyl-sn-glycero-3-phosphocholine, 1-stearoyl-2-oleoyl-sn-glycero-3-phosphocholine, 1-palmitoyl-2-myristoyl-sn-glycero-3-phosphocholine, and 1-palmitoyl-2-stearoyl-sn-glycero-3-phosphocholine.

The aforementioned phosphatidylglycerol may originate from animals or plants and is generally selected from phosphatidylglycerols purified from soybean or hen's egg yolk.

No particular limitation is imposed on the number of carbon atoms and the unsaturation degree of each of the two acyl groups in the phosphatidylglycerol. Generally, the acyl group has 10 to 22 carbon atoms and 0 to 2 unsaturated bonds. Also, the number of carbon atoms and the unsaturation degree of one acyl group are not necessarily equal to those of the other acyl group. The two acyl groups may be those having different numbers of carbon atoms, and a combination of two saturated acyl groups, a combination of a saturated acyl group and an unsaturated acyl group, or a combination of two unsaturated acyl groups may be employed. Generally, phosphatidylglycerols originating from animals and plants are in the form of a mixture of phosphatidylglycerol species bearing acyl groups having different number of carbon atoms and different unsaturation degrees.

The aforementioned phosphatidylglycerol may be a chemically synthesized product. Examples of commercial products thereof include 1,2-dimyristoyl-sn-glycero-3-phosphoglycerol sodium salt, 1,2-dimyristoyl-sn-glycero-3-phosphoglycerol ammonium salt, 1,2-dipalmitoyl-sn-glycero-3-phosphoglycerol sodium salt, 1,2-dipalmitoyl-sn-glycero-3-phosphoglycerol ammonium salt, 1,2-distearoyl-sn-glycero-3-phosphoglycerol sodium salt, 1,2-distearoyl-sn-glycero-3-phosphoglycerol ammonium salt, 1,2-dioleoyl-sn-glycero-3-phosphoglycerol sodium salt, 1,2-diercoyl-sn-glycero-3-phosphoglycerol sodium salt, and 1-palmitoyl-2-oleoyl-sn-glycero-3-phosphoglycerol sodium salt.

The phosphatidylethanolamine may originate from animals or plants and is generally selected from phosphatidylethanolamines purified from soybean or hen's egg yolk.

No particular limitation is imposed on the number of carbon atoms and the unsaturation degree of each of the two acyl groups in the phosphatidylethanolamine. Generally, the acyl group has 10 to 22 carbon atoms and 0 to 2 unsaturated bonds. Also, the number of carbon atoms and the unsaturation degree of one acyl group are not necessarily equal to those of the other acyl group. The two acyl groups may be those having different numbers of carbon atoms, and a combination of two saturated acyl groups, a combination of a saturated acyl group and an unsaturated acyl group, or a combination of two unsaturated acyl groups may be employed. Generally, phosphatidylethanolamines originating from animals and plants are in the form of a mixture of phosphatidylethanolamine species bearing acyl groups having different number of carbon atoms and different unsaturation degrees.

The aforementioned phosphatidylethanolamine may be a chemically synthesized product. Examples of commercial products thereof include 1,2-dimyristoyl-sn-glycero-3-phosphoethanolamine, 1,2-dipalmitoyl-sn-glycero-3-phosphoethanolamine, 1,2-distearoyl-sn-glycero-3-phosphoethanolamine, 1,2-dilinoleoyl-sn-glycero-3-phosphoethanolamine, and 1,2-diercoyl-sn-glycero-3-phosphoethanolamine.

The aforementioned glycerophospholipid is preferably dissolved or dispersed in the buffer for the reaction system. A scarcely soluble glycerophospholipid may be dissolved or dispersed through ultrasonication. Alternatively, a surfactant may be added to dissolve such a glycerophospholipid.

No particular limitation is imposed on the surfactant generally employed above, so long as it can solubilize the lipid. Examples of preferred surfactants include sucrose fatty acid esters such as sucrose monolaurate; alkylglycosides such as lysophosphatidylcholine, octylglucoside, and dodecylmaltoside; and dextran sulfate.

The analyte of the present inventionis an antiphospholipid antibody or a prostate-specific antigen Examples of the antiphospholipid antibody include an anti-syphilis phospholipid antibody, which emerges in blood through infection with syphilis, and an antiphospholipid antibody, which emerges in blood through infection with antiphospholipid antibody syndrome (i.e., an autoimmune disease).

In the reagent of the present invention, when the analyte is an antibody, the antibody is subjected to immune reaction with an antigen, whereas when the analyte is an antigen, the antigen is subjected to immune reaction with an antibody. Thus, the reagent of the present invention generally contains an antigen or antibody which reacts with the target analyte via immune reaction. When the analyte is an antiphospholipid antibody, phospholipid is used as an antigen. When the analyte is prostate specific antigen, an anti-prostate specific antigen antibody is used as an antibody.

As the phospholipid serving as the phospholipid antigen, three phospholipids, i.e., cardiolipin, phosphatidylcholine, and cholesterol, are generally employed. However, it is not necessarily the case that all three phospholipids are contained in the reagent, and any of the three can be selected depending on the disease to be detected. For example, the reagent for detecting antiphospholipid antibody syndrome (i.e., an autoimmune disease) contains at least cardiolipin, and the anti-syphilis phospholipid antibody assaying reagent contains at least cardiolipin and phosphatidylcholine. Phosphatidylcholine and cholesterol are often used with cardiolipin as a mixture for the enhancement of specificity and sensitivity. The proportions by weight among three phospholipids; i.e., cardiolipin : phosphatidylcholine : cholesterol are preferably about 1 : (3 to 30) : (0 to 10), more preferably about 1 : (3 to 30) : (0.5 to 10). However, the proportions are not limited thereto and are appropriately adjusted depending on the purpose of the reagent.

The phospholipid may be obtained from animals and plants or chemically synthesized, and the production method is appropriately chosen depending on the purpose. Generally, cardiolipin which is extracted and purified from cow's heart, phosphatidylcholine which is extracted and purified from hen's egg yolk, and cholesterol which is extracted from wool, and all of these compounds which are synthesized can be used. Alternatively, these phospholipids employed in the invention may be synthesized products or commercial products.

Before use, the aforementioned antigens such as a phospholipid antigen or the aforementioned antibodies such as an anti-prostate specific antigen antibody are generally dispersed in an appropriate solution. No particular limitation is imposed on the solution, and phosphate buffer, Tris-HCl buffer, glycine buffer, etc. may be employed. In dispersing such an antigen in the solution, the antigen is dispersed by causing it to be supported on an insoluble carrier.

In the present invention, as the insoluble carrier a latex carrier formed of a synthetic polymer, which can be mass-produced on an industrial scale, is used. Examples of the synthetic polymer include polystyrene, styrene-sulfonic acid copolymer, styrene-methacrylic acid copolymer, acrylonitrile-butadiene-styrene copolymer, vinyl chloride-acrylate ester copolymer, and vinyl acetate-acrylate ester copolymer. Of these, polystyrene and styrene-sulfonic acid copolymer are particularly preferred, in view of the fact that these polymers efficiently adsorb phospholipid and can stably maintain biological activity during a long-term storage period. The mean particle size of the insoluble carrier generally employed, which varies depending on the determination method and the measurement apparatus, is 0.1 to 1.0 µm as determined by means of a transmission electron microscope, preferably 0.1 to 0.5 µm.

No particular limitation is imposed on the method for causing the phospholipid antigen to be supported on the insoluble carrier. For example, the phospholipid antigen is caused to be supported on the insoluble carrier via physical and/or chemical bonding by using a conventionally known technique.

In a procedure of causing the phospholipid antigen to be physically supported on the insoluble carrier, a latex having a suitable particle size is mixed with a phospholipid dissolved in a suitable solvent for example ethanol (i.e., phospholipid latex mixture liquid) under stirring (sensitization step), and after passage over a specific period of time, the mixture is treated with a solution containing protein, sugar, peptide, etc. (blocking step), followed by dispersing it in an appropriate solvent.

Examples of the solvent in which the latex is dispersed include phosphate buffer, Tris-HCl buffer, and glycine buffer.

Examples of the sample analyzed through these test methods include blood samples, serum samples, plasma samples, and cerebrospinal fluid samples, which possibly contain the aforementioned analyte.

In the measurement procedure of the present invention, the degree of agglutination occurring from the immune reaction between the above-produced reagent and the analyte (e.g., an antiphospholipid antibody) present in the sample is optically measured, to thereby determine the amount of analyte (e.g., an antiphospholipid antibody) present in the sample.

The agglutination degree is optically measured through a known technique. Examples of the technique include turbidimetry in which formation of agglutination is measured as an increase in turbidity; a method in which formation of agglutination is measured as a change in particle size distribution or mean particle size; and integrating-sphere optical turbidimetry in which a change in forward-scattered light attributed to formation of agglutination is measured by means of an integrating sphere, and the ratio of the change to the transmitted light intensity is analyzed. In any of the measurement techniques, at least two measurements are obtained at different points in time, and the degree of agglutination is obtained on the basis of the rate of increase in the measurements between the time points (rate assay). Alternatively, the measurement is performed at a certain point in time (typically, a conceivable end point of reaction), and the degree of agglutination is obtained on the basis of the measurement (end point assay). From the viewpoints of simplicity and speed of the measurement, the rate assay based on turbidimetry is preferably performed. In the optical measurement, there may be employed an optical instrument which can detect scattered light intensity, transmitted light intensity, absorbance, etc.; in particular, a generally employed automated analyzer. The measurement may be performed at a wavelength of 250 to 1,000 nm, preferably 540 to 800 nm.

No particular limitation is imposed on the reaction temperature, so long as the aforementioned immune reaction occurs. The immune reaction is preferably performed at a constant temperature of 10 to 50°C, more preferably 10 to 40°C. The reaction time is appropriately adjusted.

No particular limitation is imposed on the reaction medium (liquid) of the reaction system where the immune reaction is performed, so long as the medium is an aqueous solution having a property which can satisfy physiological conditions under which the immune reaction occurs. Examples of the medium include phosphate buffer, citrate buffer, glycine buffer, Tris buffer, and Good's buffer. The reaction medium preferably has a pH of 5.5 to 8.5, more preferably 6.5 to 8.0. If needed, the reaction medium may further contain a stabilizer such as bovine serum albumin or sucrose; an antiseptic such as sodium azide; a salt-concentration-controlling agent such as sodium chloride; etc.

In order to enhance assay sensitivity and/or promote antigen-antibody reaction, an aqueous polymer may be added to the antiphospholipid antibody assay reagent of the present invention. Examples of the aqueous polymer include pullulan and polyvinylpyrrolidone. Of these, polyvinylpyrrolidone is particularly preferably added to the antiphospholipid antibody assay reagent of the present invention. Examples

The present invention will next be described in detail by way of examples, which should not be construed as limiting the present invention thereto.

### Example 1

### 1) Preparation of lipid antigen liquid

2 mL of cardiolipin solution in ethanol (5 mg/mL, product of Sigma), 10 mL of phosphatidylcholine (COATSOME NC-50, product of NOF Corporation) solution in ethanol (10 mg/mL), and 3 mL of cholesterol (product of Nacalai Tesque) solution in ethanol (10 mg/mL) were mixed together, to thereby prepare a lipid antigen liquid.

### 2) Production of latex particles

1,100 g of distilled water, 200 g of styrene, 0.2 g of sodium styrenesulfonate, and an aqueous solution prepared by dissolving 1.5 g of potassium persulfate in 50 g of distilled water were fed to a glass reactor (capacity: 2 L) equipped with a stirrer, a reflux condenser, a temperature sensor, a nitrogen conduit, and a jacket. The atmosphere of the reactor was changed to nitrogen, and then the mixture in the reactor was allowed to polymerize at 70°C under stirring for 48 hours.

After completion of polymerization, the reaction mixture was filtered through filter paper, to thereby recover latex particles. The mean particle size of the latex particles was determined by imaging the latex particles by means of a transmission electron microscope (JEM-1010, product of JEOL Ltd.) with 10,000-fold magnification. The image analysis was performed with respect to at least 100 particles. Thus, latex A having a mean particle size of 0.40 µm was produced.

### 3) Preparation of lipid antigen-sensitized latex reagent

100 µL of the latex A (solid content: 10 wt.%) produced in 2) above was gently stirred and maintained at 37°C. To the latex A, 330 µL of the aforementioned lipid antigen liquid was added in a batch manner, and the mixture was gently stirred at 37°C for 2 hours. Subsequently, 2 mL of 100-mmol/L phosphate buffered saline (100-mmol/L phosphate buffer (pH: 7.4) and 0.9 wt.% of NaCl; hereinafter abbreviated as PBS) containing 1 wt.% bovine serum albumin (hereinafter abbreviated as BSA) (Fraction V, reagent grade, product of Millipore) was added in a batch manner, and the mixture was further stirred at 37°C for one hour. This product was centrifuged, to thereby remove the supernatant, and the precipitated latex was suspended again in PBS containing 1 wt.% BSA. The purification procedure was repeated. Finally, the thus-purified latex was suspended in 100-mmol/L phosphate buffer (pH: 7.4) (hereinafter abbreviated as PB) (4 mL) containing 1 wt.% BSA, 7.5 wt.% choline chloride, 0.14 wt.% EDTA·2Na, and 0.1 wt.% sodium azide, to thereby produce a latex reagent.

### 4) Preparation of sample-diluent

BSA, pullulan (molecular weight: 200,000, product of Hayashibara), sodium azide, and an egg-yolk-derived phosphatidylglycerol (COATSOME NG-50LS, product of NOF Corporation) were added to 50-mmol/L phosphate buffer (pH: 7.4) such that the amounts of the ingredients were adjusted to 1 wt.%, 1.0 wt.%, 0.1 wt.%, and 0.17 wt.%, respectively, and the mixture was stirred. Under ice-cooling conditions, the mixture was subjected to ultrasonication for 30 minutes or longer by means of an ultrasonic crusher (power 20%, 0.25-inch microchip) until the mixture became a transparent solution, to thereby prepare a sample-diluent.

### Example 2

The procedure of "4) Preparation of sample-diluent" of Example 1 was repeated, except that the egg-yolk-derived phosphatidylglycerol concentration was adjusted to 0.085 wt.%.

### Example 3

The procedure of "4) Preparation of sample-diluent" of Example 1 was repeated, except that the egg-yolk-derived phosphatidylglycerol concentration was adjusted to 0.043 wt.%.

### Example 4

The procedure of "4) Preparation of sample-diluent" of Example 1 was repeated, except that a synthetic phosphatidylglycerol-1,2-dimyristoyl-sn-glycero-3-phosphoglycerol sodium salt (COATSOME MG-4040, product of NOF Corporation)-was used at a concentration of 0.043 wt.%, instead of the egg-yolk-derived phosphatidylglycerol.

### Example 5

The procedure of "4) Preparation of sample-diluent" of Example 1 was repeated, except that an egg-yolk-derived phosphatidylethanolamine (COATSOME NE-50, product of NOF Corporation) was used at a concentration of 0.085 wt.%, instead of the egg-yolk-derived phosphatidylglycerol.

### Example 6

The procedure of "4) Preparation of sample-diluent" of Example 1 was repeated, except that a synthetic phosphatidylcholine-1,2-dimyristoyl-sn-glycero-3-phosphocholine (COATSOME MC-4040, product of NOF Corporation)-was used at a concentration of 0.043 wt.%, instead of the egg-yolk-derived phosphatidylglycerol, and that lysophosphatidylcholine (COATSOME MC-40H, product of NOF Corporation) serving as a surfactant was used at a concentration of 0.014 wt.%.

### Example 7

The procedure of "4) Preparation of sample-diluent" of Example 1 was repeated, except that a synthetic phosphatidylcholine-1-palmitoyl-2-stearoyl-sn-glycero-3-phosphocholine (COATSOME MC-6080, product of NOF Corporation)-was used at a concentration of 0.043 wt.%, instead of the egg-yolk-derived phosphatidylglycerol, and that lysophosphatidylcholine (COATSOME MC-40H, product of NOF Corporation) serving as a surfactant was used at a concentration of 0.014 wt.%.

### Example 8

The procedure of "4) Preparation of sample-diluent" of Example 1 was repeated, except that the egg-yolk-derived phosphatidylglycerol concentration was adjusted to 0.021 wt.%, and an egg-yolk-derived phosphatidylethanolamine was used at a concentration of 0.021 wt.%.

### Comparative Example 1

The procedure of "4) Preparation of sample-diluent" of Example 1 was repeated, except that no glycerophospholipid was added.

### Example 9

### 1) Production of latex particles

1,100 g of distilled water, 200 g of styrene, 0.2 g of sodium styrenesulfonate, and an aqueous solution prepared by dissolving 1.5 g of potassium persulfate in 50 g of distilled water were fed to a glass reactor (capacity: 2 L) equipped with a stirrer, a reflux condenser, a temperature sensor, a nitrogen conduit, and a jacket. The atmosphere of the reactor was changed to nitrogen, and then the mixture in the reactor was allowed to polymerize at 70°C under stirring for 48 hours.

After completion of polymerization, the reaction mixture was filtered through filter paper, to thereby recover latex particles. The mean particle size of the latex particles was determined by imaging the latex particles by means of a transmission electron microscope (JEM-1010, product of JEOL Ltd.) with 10,000-fold magnification. The image analysis was performed with respect to at least 100 particles. Thus, latex B having a mean particle size of 0.40 µm was produced.

### 2) Preparation of anti-PSA-antibody-sensitized latex reagent

Each of latex B (solid content: 10% (w/v)) and anti-PSA antibody 63291 was diluted with 20-mmol/L glycine buffer (pH: 9.0), to thereby prepare 1% latex liquid and 0.4-mg/mL antibody liquid. These two liquids were mixed at a ratio of 1 : 1 (1 vol. + 1 vol.), and the mixture was stirred for about one hour. To 2 parts by volume of the resultant mixture, 0.1 parts by volume of a blocking liquid (10 wt.% BSA) was added, and the mixture was stirred for about one hour. Subsequently, the mixture was dialyzed against 5-mmol/L MOPS (pH: 7.0) solution. The resultant solution was diluted so that the absorbance index after dialysis was adjusted to 3 Abs/mL (600 nm), to thereby prepare anti-PSA antibody-sensitized latex solution a.

Also, the procedure of the above preparation of anti-PSA antibody-sensitized latex solution a was repeated, except that latex B (solid content: 10% (w/v)) and anti-PSA antibody 63251 were used, to thereby prepare anti-PSA antibody-sensitized latex solution b.

The thus-prepared latex solutions a and b were mixed at a ratio of 1 : 1 (1 vol. + 1 vol.), to thereby prepare an anti-PSA-antibody-sensitized latex reagent.

### 3) Preparation of sample-diluent

BSA, potassium chloride, Polyvinylpyrrolidone K90 (product of Wako Pure Chemical Industries, Ltd.), and an egg-yolk-derived phosphatidylglycerol (COATSOME NG-50LS, product of NOF Corporation) were added to 30-mmol/L HEPSE buffer (pH: 7.0) such that the amounts of the ingredients were adjusted to 0.1 wt.%, 0.5 mol/L, 0.3%, and 0.115 wt.%, respectively, and the mixture was stirred. Then, the mixture was subjected to ultrasonication for 30 minutes or longer by means of an ultrasonic crusher until the mixture became a transparent solution, to thereby prepare a sample-diluent.

### Comparative Example 2

The procedure of "3) Preparation of sample-diluent" of Example 9 was repeated, except that no glycerophospholipid was added.

The thus-produced reagents (reagents) of Examples 1 to 9 and Comparative Examples 1 and 2 were tested, and the results are as follows.

### (Test 1)

### 1) Fractionation of endogenous lipoprotein

Fractions containing high-density lipoprotein (hereinafter abbreviated as HDL) were recovered through potassium bromide density-gradient centrifugation.

In a specific procedure, 1.21-g/mL potassium bromide solution was dispensed into ultracentrifugation tubes (capacity: 50 mL) at 12 mL/tube. To each tube, 10 mL of syphilis-negative serum was slowly dispensed such that the serum was not intermingled with potassium bromide solution, to thereby form two strata. Then, in a similar manner, 12 mL of purified water was slowly dispensed such that water was not intermingled with the serum, to form another stratum thereon.

The tubes were subjected to centrifugation by means of an ultra-centrifuge at 4°C and 44,000 rpm for 20 hours.

An injection needle was connected to a tube made of Teflon (registered trademark) and employed so that a solution can be sampled through suction by means of a peristaltic pump connected thereto. Specifically, the injection needle was slowly inserted into the bottom of each of the ultracentrifugation tubes after ultracentrifugation, and serum having potassium bromide density gradient was recovered through suction by means of the peristaltic pump. An aliquot (0.7 mL) was sampled as a fraction from each tube, and 43 fractions were recovered in total.

Through measurement of the HDL cholesterol levels of the 43 fractions, HDL was found to be contained in fractions No. 5 to No. 15. Fractions No. 9 to No. 13, corresponding to the highest HDL cholesterol level region, were combined and dialyzed against physiological saline in order to remove potassium bromide. After dialysis, the HDL cholesterol level was determined by use of Cholestest (registered trademark) N HDL (product of Sekisui Medical Co., Ltd.), and the level was found to be 40 mg/dL.

Thus, an HDL-containing physiological saline solution was produced.

### 2) Assay samples

A syphilis antiphospholipid antibody-positive sample having an antibody titer of 120 R.U. was stepwise diluted with the HDL-containing physiological saline obtained in 1) of Test 1 above, physiological saline, or serum. The unit R.U. is a unit of syphilis-positive antibody titer. A titer of 1 R.U. corresponds to unity in the RPR card test. When the titer is 1 R.U. or higher, the sample is diagnosed as syphilis positive. When the international standard sample is assayed, a titer of 1 R.U. corresponds to 0.4 IU.

### 3) Measurement of agglutination amount

Agglutination amount was determined by means of a biochemical auto-analyzer (Hitachi 7180). 180 µL of the sample-diluent prepared in Example 2 or Comparative Example 1 and 20 µL of each sample produced in 2) of Test 1 were mixed together in a cell of the biochemical auto-analyzer. The mixture was incubated at 37°C for 5 minutes. Subsequently, 60 µL of the latex reagent produced in 1) of Example 1 was added to and mixed with the incubate, and the mixture was incubated at 37°C for 5 minutes. The absorbance of the sample at a measurement wavelength of 700 nm was measured immediately after addition of the latex reagent and five minutes after the addition, and the difference between two measurements was calculated by means of the auto-analyzer (hereinafter represented by ΔAbs×10,000). The difference in absorbance corresponds to the amount of agglutination increased by immune reaction.

### 4) Results

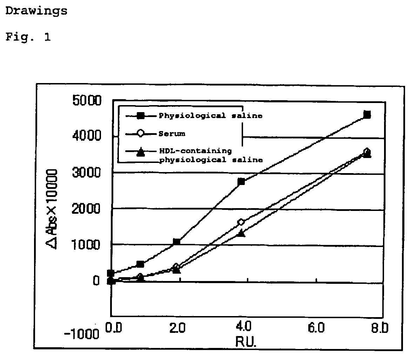

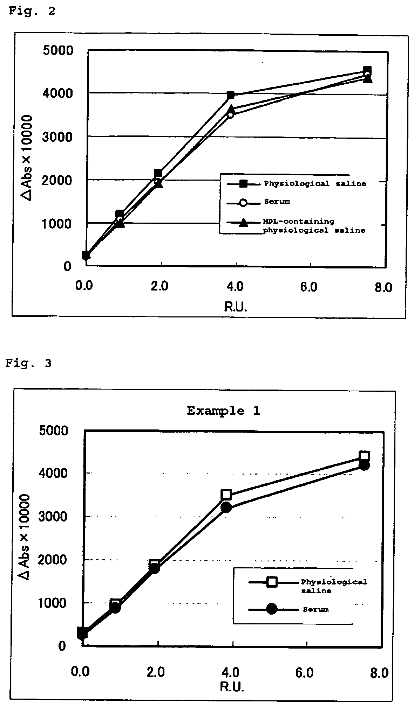

Figs. 1 and 2 show the results.

As shown in Fig. 1 and 2, in Comparative Example 1, a drop in absorbance was observed when the HDL-containing physiological saline was used in a manner similar to the case in which serum was used. Thus, HDL present in serum was thought to be an interference component causing a drop in absorbance. In contrast in Example 2 in which a glycerophospholipid was added, a drop in absorbance as observed in Comparative Example 1 was suppressed, when the sample was diluted with the HDL-containing physiological saline or serum, and the obtained absorbance was almost equivalent to that obtained by a similar sample diluted with HDL-free physiological saline. Therefore, the glycerophospholipid added in Example 2 was found to suppress interference caused by HDL, to thereby suppress a drop in absorbance.

### (Test 2)

### 1) Assay samples

A syphilis antiphospholipid antibody-positive sample having an antibody titer of 120 R.U. was stepwise diluted with physiological saline or serum.

### 2) Measurement of agglutination amount

Agglutination amount was determined by means of a biochemical auto-analyzer (Hitachi 7180). 180 µL sample-diluent prepared in any of Examples 1 to 8 and Comparative Example 1 and 20 µL of each sample () produced in 1) of Test 1 were mixed together in a cell of the biochemical auto-analyzer. The mixture was incubated at 37°C for 5 minutes. Subsequently, 60 µL of the latex reagent produced in 1) of Example 1 was added to and mixed with the incubate, and the mixture was incubated at 37°C for 5 minutes. The absorbance of the sample at a measurement wavelength of 700 nm was measured immediately after addition of the latex reagent and five minutes after the addition, and the difference between two measurements was calculated by means of the auto-analyzer (hereinafter represented by ΔAbs×10,000). The difference in absorbance corresponds to the amount of agglutination increased by immune reaction.

### 3) Results

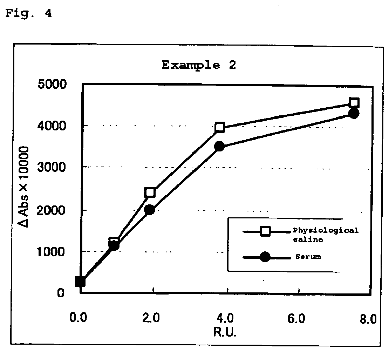

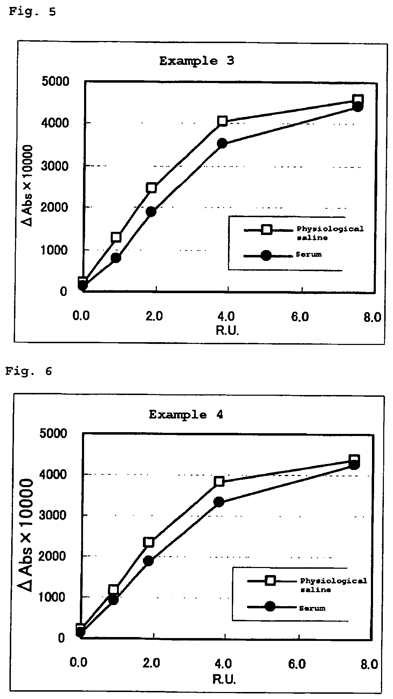

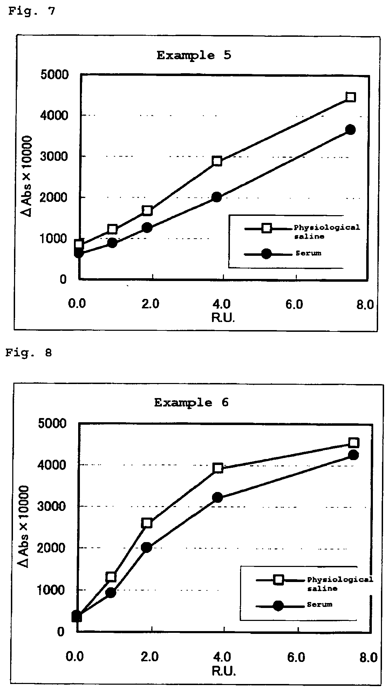

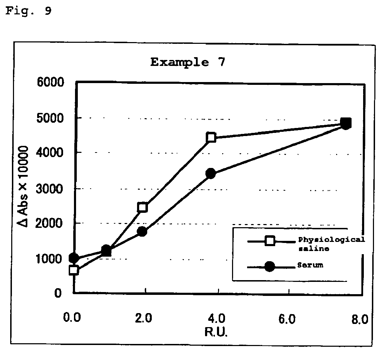

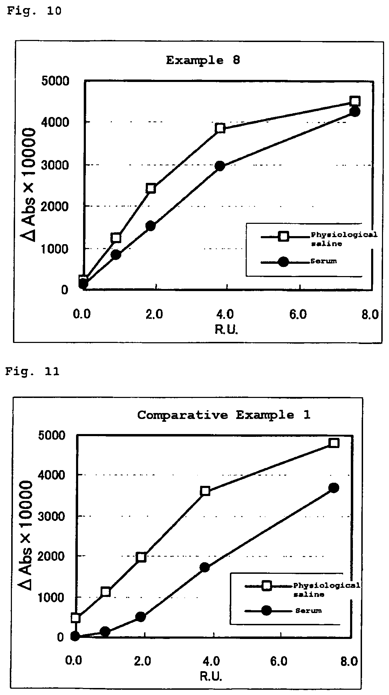

Figs. 3 to 11 show the results.

In Examples 1 to 8, the difference in absorbance at any antibody concentration decreased when the sample was diluted with physiological saline or serum, as compared with Comparative Example 1. Thus, the drop in absorbance, which would otherwise be caused by an interference component present in serum, was found to be mitigated through addition of any of the glycerophospholipids.

### (Test 3)

### 1) Assay samples

A sample having a PSA level of 9.5 ng/mL was diluted with physiological saline or female serum, to thereby provide samples of Test 3.

### 2) Measurement of agglutination amount

Agglutination amount was determined by means of a biochemical auto-analyzer (Hitachi 7180). 90 µL of sample-diluent prepared in any of Example 9 and Comparative Example 2 and 10.8 µL of each sample produced in 1) of Test 1 were mixed together in a cell of the biochemical auto-analyzer. The mixture was incubated at 37°C for 5 minutes. Subsequently, 90 µL of the latex reagent produced in 1) of Example 9 was added to and mixed with the incubate, and the mixture was incubated at 37°C for 5 minutes. The absorbance of the sample at a measurement wavelength of 800 nm was measured immediately after addition of the latex reagent and five minutes after the addition, and the difference between two measurements was calculated by means of the auto-analyzer (hereinafter represented by ΔAbs×10,000). The difference in absorbance corresponds to the amount of agglutination increased by immune reaction.

### 3) Results

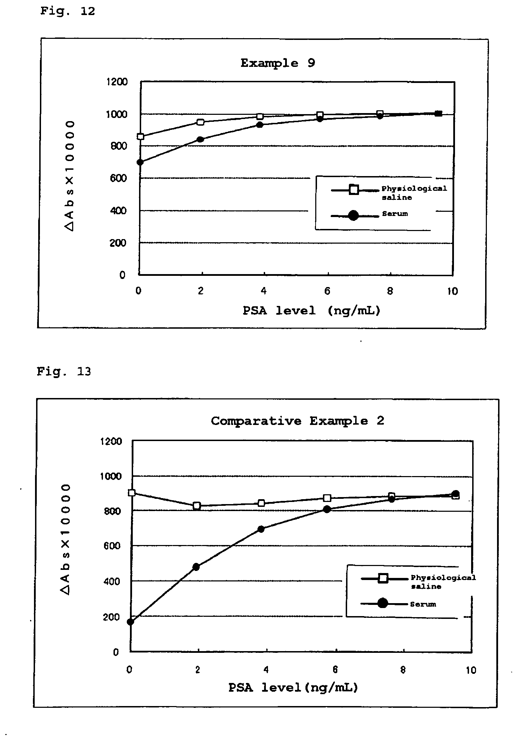

Figs. 12 and 13 show the results.

In Example 9, the difference in absorbance at any PSA level decreased when the sample was diluted with physiological saline or serum, as compared with Comparative Example 2.

Thus, the drop in absorbance, which would otherwise be caused by an interference component present in serum, was found to be mitigated through addition of any of the glycerophospholipids.

### Industrial Applicability

The present invention discloses a method for avoiding any interference effect caused by an endogenous lipoprotein in an assay of an analyte in blood through immune reaction by use of a reagent. When an assay is performed by use of an antiphospholipid antibody assay reagent, which is interfered by an endogenous lipoprotein, the present invention can avoid the interference effect, to thereby more accurate measurements can be obtained.

## Claims

1. A reagent for assaying an antibody or an antigen serving as an analyte in blood by immune reaction with an antigen when the analyte is an antibody or by immune reaction with an antibody when the analyte is an antigen, wherein the antigen, when the analyte is an antibody, or the antibody, when the analyte is an antigen, is supported on a latex carrier formed of a synthetic polymer, and the amount of analyte is measured by optically measuring the degree of agglutination formed by the immune reaction between the latex carrier and the analyte,
wherein a glycerophospholipid in the form of solution or dispersion is incorporated into the immune reaction system,
wherein the glycerophospholipid is one or more species selected from the group consisting of phosphatidylcholine, phosphatidylglycerol, and phosphatidylethanolamine, and
wherein the analyte is an antiphospholipid antibody or a prostate specific antigen.

2. The reagent according to claim 1, wherein the antiphospholipid antibody serving as an analyte is an anti-syphilis phospholipid antibody generated in blood through infection with syphilis.

3. The reagent according to claim 1 or 2, wherein the antiphospholipid antibody serving as an analyte is an antiphospholipid antibody generated in blood through antiphospholipid antibody syndrome, which is an autoimmune disease.

## Patentansprüche

1. Reagenz zur Untersuchung eines Antikörpers oder eines Antigens, das als Analyt im Blut dient, durch eine Immunreaktion mit einem Antigen, wenn der Analyt ein Antikörper ist, oder durch eine Immunreaktion mit einem Antikörper, wenn der Analyt ein Antigen ist, wobei das Antigen, wenn der Analyt ein Antikörper ist, oder der Antikörper, wenn der Analyt ein Antigen ist, von einem Latexträger, der aus einem synthetischen Polymer gebildet ist, getragen wird, und die Menge des Analyten gemessen wird, indem der Grad der Agglutination, die durch die Immunreaktion zwischen dem Latexträger und dem Analyten gebildet wurde, optisch gemessen wird, wobei ein Glycerophospholipid in Form einer Lösung oder einer Dispersion in das Immunreaktionssystem aufgenommen wird, wobei das Glycerophospholipid eine oder mehrere Arten sind, ausgewählt aus der Gruppe, bestehend aus Phosphatidylcholin, Phosphatidylglycerol und Phosphatidylethanolamin, und wobei der Analyt ein Antiphospholipid-Antikörper oder ein Prostata-spezifisches Antigen ist.

2. Reagenz nach Anspruch 1, wobei der Antiphospholipid-Antikörper, der als Analyt dient, ein anti-Syphilis-Phospholipid-Antikörper ist, der im Blut durch eine Infektion mit Syphilis erzeugt wird.

3. Reagenz nach Anspruch 1 oder 2, wobei der Antiphospholipid-Antikörper, der als Analyt dient, ein Antiphospholipid-Antikörper ist, der im Blut durch das Antiphospholipid-Antikörper-Syndrom, welches eine Autoimmunerkrankung ist, erzeugt wird.

## Revendications

1. Réactif pour doser un anticorps ou un antigène servant d'analyte dans le sang par réaction immunologique avec un antigène quand l'analyte est un anticorps ou par réaction immunologique avec un anticorps quand l'analyte est un antigène,
dans lequel l'antigène, quand l'analyte est un anticorps, ou l'anticorps, quand l'analyte est un antigène, est supporté sur un support en latex formé d'un polymère synthétique, et la quantité d'analyte est mesurée par mesure optique du degré d'agglutination formée par la réaction immunologique entre le support en latex et l'analyte,
dans lequel un glycérophospholipide sous la forme d'une solution ou d'une dispersion est incorporé dans le système réactionnel immunologique,
dans lequel le glycérophospholipide est une ou plusieurs espèces choisies dans l'ensemble constitué par la phosphatidylcholine, le phosphatidylglycérol, et la phosphatidyléthanolamine, et
dans lequel l'analyte est un anticorps anti-phospholipide ou un antigène spécifique de la prostate.

2. Réactif selon la revendication 1, dans lequel l'anticorps anti-phospholipide servant d'analyte est un anticorps anti-phospholipide de la syphilis généré dans le sang par l'intermédiaire d'une infection par la syphilis.

3. Réactif selon la revendication 1 ou 2, dans lequel l'anticorps anti-phospholipide servant d'analyte est un anticorps anti-phospholipide généré dans le sang par l'intermédiaire du syndrome des anticorps anti-phospholipide, qui est une maladie auto-immune.
